# EUROPEAN PATENT SPECIFICATION

(11) **EP 1 875 929 B1**
(45) Date of publication and mention of the grant of the patent: **10.11.2010**
(21) Application number: 07111970.5
(22) Date of filing: 06.07.2007
(51) Int. Cl.: A61L 17/04

(54) **Suture with filaments formed of polyether-ketone variant**
Naht mit aus einer Polyether-Keton-Variante geformten Fasern
Suture avec filaments formés de variante de polyéther-cétoniques

(30) Priority: 07.07.2006 US 819001 P; 01.05.2007 US 915296 P
(43) Date of publication of application: 09.01.2008
(73) Proprietor: Arthrex, Inc., Naples, FL 34108-1945 (US)
(72) Inventor: Schmieding, John, W., Naples, FL 34109 (US)
(74) Representative: Long, Giorgio

(56) References cited:
- EP-A- 1 293 218
- EP-A- 1 857 073
- DATABASE EMBASE [Online] ELSEVIER SCIENCE PUBLISHERS, AMSTERDAM, NL; November 2000 (2000-11), ESCHBACH L: "Nonresorbable polymers in bone surgery" XP002490479 Database accession no. EMB-2001164508 & INJURY 200011 GB, vol. 31, no. SUPPL. 4, November 2000 (2000-11), pages D22-D27, ISSN: 0020-1383

## Description

### FIELD OF THE INVENTION

The present invention relates to high strength surgical suture materials, and more particularly, to a braided suture having filaments formed of a polyether-ketone variant.

### BACKGROUND OF THE INVENTION

Sutures are categorized into several groups - absorbable and non-absorbable; monofilament and multifilament; natural and synthetic. Absorbable sutures degrade by two major mechanisms: (i) sutures of biological origin such as surgical gut are gradually digested by tissue enzymes; and (ii) sutures manufactured from synthetic polymers are principally broken down by hydrolysis in tissue fluids. Non-absorbable sutures, made from a variety of non biodegradable materials, are ultimately encapsulated or walled off by fibroblasts.

A monofilament suture is made of a single strand. A multifilament suture consists of several filaments twisted or braided together. A multifilament gives good handling and tying qualities. Examples of natural sutures are catgut and silk. Synthetic suture material comprises polyglycolic acid (dexon), polyglactin (vicryl), polydioxone (PDS), polyglyconate (Maxon), polyamide (nylon), polyester (dacron), polypropylene (prolene).

Suture strength is an important consideration in any surgical suture material. One of the strongest materials currently formed into elongated strands is an ultrahigh molecular weight long chain polyethylene (UHMWPE), typically used for fishing line and the like, which is sold under the trade names Dyneema and Spectra. This material is much stronger than ordinary surgical suture, however, it does not have acceptable knot tie down characteristics itself for use in surgical applications. However, an exceptionally strong suture with acceptably handling characteristics can be formed by braiding fibers of UHMWPE with polyester, as disclosed and claimed in U.S. Pat. No. 6,716,234.

Although not previously recognized in the art, a material which can be used in a high strength suture, either alone or in combination with other materials such ultrahigh molecular weight polyethylene, is PEEK. PEEK is an abbreviation for polyetherether-ketone, a high performance engineering thermoplastic. It is a semicrystalline, linear aromatic polymer and is widely regarded as the highest performance thermoplastic material currently available. PEEK has excellent chemical resistance, very low moisture absorption, inherently good wear and abrasion resistance, and is unaffected by continuous exposure to hot water or steam. The advantages of PEEK are described in a white paper entitled, "New Materials in Sports Medicine," Arthrex, Inc., 2005. Advantages of PEEK generally known in the art are discussed below. PEEK is sold under the trademark PEEK™ by Victrex PLC;
http://www.victrex.com/en/index.php; 3 Caledon Court - Suite A, Greenville, SC 29615, USA.

PEEK polymer has excellent friction and wear properties which are optimized in the specially formulated tribological grades 450FC30 and 150FC30. These materials exhibit outstanding wear resistance over wide ranges of pressure, velocity, temperature and counterfacial roughness. PEEK polymer has excellent resistance to a wide range of chemical environments, even at elevated temperatures. PEEK grades offer chemical and water resistance similar to PPS (polyphenylene sulfide), but can operate at higher temperatures. It can be used continuously to 480°F (250°C) and in hot water or steam without permanent loss in physical properties. The only common environment which dissolves PEEK polymer is concentrated sulphuric acid. For hostile environments, PEEK is a high strength alternative to fluoropolymers.

PEEK polymer and compounds are not chemically attacked by water or pressurized steam. Components which are constructed from these materials retain a high level of mechanical properties when continuously conditioned in water at elevated temperatures and pressures.

Other properties of PEEK are included in the following Table:

**Table 1: Property Table**

| **Properties** | **ASTM or Unit** | **PEEK™** |
|---|---|---|
| Specific Gravity | D792 | 1.30~1.32 |
| Elongation % | D638 | 20~60 |
| Tensile Strength (psi) | D638 | 14,065~14,500 |
| Flexural Strength (psi) | D790 | 24,650 |
| Compressive Strength | D695 | 17,110 |
| Tensile Elastic Modulus (Young's Modulus) (psi) | D638 | 522,000 |
| Flexural Modulus (psi) | D790 | 580,000~594,500 10³MPa(10³kgf/m²) |
| Hardness Durometer Shore D | D636 | D85~86 |
| Coefficient of Friction on steel | D1984 | 0.18 (Dynamic) |
| Impact Strength IZOD 73°F/23°C, notched ft/lbs/in | D256 | 1.6 |
| Melting Point Melting Point | °C (°F) | 340 (644) |
| Upper Service Temperature(20,000h) | °C (°F) | 260 (500) |
| Flame Rating | UL 94 | V-0 |
| Thermal Conductivity | BTU/hr/ft²/deg F in | 1.73 |
| Linear Coefficient of Thermal Expansion | D696 | 2.6 10⁻⁵ °F-1 |
| Dielectric Constant | 50Hz-10kHz | 3.20~3.30 |
| Dielectric Strength 10 mil film | D149 | >500 |
| Volume Resistivity ohm-cm | D257 | 4.9 x 10¹⁶ |
| Surface Resistivity ohm/sq. | D257 | 2.0 x 10¹⁶ |
| Chemical/Solvent Resistance | D543 | Excellent |
| Water Absorption, 24h,% | D570 | 0.5 |
| Refractive Index | | 2.15 |
| Limiting Oxygen Index % | D2863 | 24 |

PEEK has been used widely in aerospace, automotive, electronics, defense, food processing, and medical applications. Such use is the result of the properties of PEEK - chemical resistance; high strength for application/part longevity; inherent purity and extremely inert for sterile environments; outstanding autoclavability; impact and wear resistance; and processing and design flexibility.

It has not been previously contemplated in the art to provide PEEK fibers (fibers made in whole or in part of PEEK) in a suture to improve the handling characteristics and tissue compatibility of a suture. However, high strength sutures incorporating PEEK fibers would add to the surgical arts, particularly in areas of orthopedic surgery. Most beneficial would be high strength sutures with PEEK that manifest acceptable knot tie-down characteristics and handling. Also beneficial would be sutures with PEEK that manifest high mechanical strength, excellent stress cracking resistance and hydrolytic stability in the presence of hot water, steam, solvents and chemicals.

Other materials which have better material properties than the ordinary surgical suture material are members of the polyether ketone family - polyetherketone (PEK), polyetherketoneketone (PEKK), and other polymer variants of ether and ketone.

The ideal suture for use in surgery would be one that is biologically inert and causes no tissue reaction. To avoid an excess tissue reaction, a surgeon should choose the smallest diameter suture with sufficient strength for the task at hand. Further, the suture must be easy for the surgeon to handle and knot reliably. The suture must have stiffness substantially similar to bone. Accordingly, the need exists for a suture material that provides high strength, is easy to handle and to knot reliably, and causes no tissue reaction.

### SUMMARY OF THE INVENTION

The present invention overcomes the disadvantages of the prior art and fulfills the needs noted above by providing high strength suture materials, particularly, braided suture blends formed with polyether ketone variants having surgically-useful qualities, including knot tie down characteristics and handling.

The suture features PEEK fibers (fibers made in whole or in part of PEEK filaments), optionally braided with UHMWPE for strength or enhancement fibers to improve handling characteristics and tissue compatibility, for example, of the high strength suture material. Fibers of this material are much stronger than those used to make ordinary surgical suture. Enhancements in tissue compatibility include improving compliance by allowing the ends of the suture to be cut close to the knot without concern for deleterious interaction between the ends of the suture and surrounding tissue. Other enhancements include incorporating visible traces into the finished suture.

The high strength sutures of the present invention preferably are formed by braiding. Plain hollow braids of PEEK are most preferred, though the various other types of braiding can be used. One or more enhancement fibers can be blended into the braid. The sutures also can include a core, preferably formed of twisted fibers. In a preferred embodiment, the core includes, or is made exclusively of, PEEK. Other core materials can be used in place of or in addition to PEEK, for example, ultrahigh molecular weight polyethylene.

In another embodiment, the suture features a jacket made of a blend of ultrahigh molecular weight polymer fibers, for example, ultrahigh molecular weight polyethylene, and one or more thermoplastic fiber, preferably a member of the polyether-ketone family. The UHMWPE provides strength. The polyether-ketone provides improved mechanical properties including tie down properties. Handling properties of the high strength suture may be enhanced using various materials to coat the suture.

As a further enhancement, fibers of a contrasting color may be added to the braided threads to enhance visibility and to make the suture more discernable during surgical procedures. The colored fibers preferably are dyed filaments. Natural fibers, such as silk, and some synthetic fibers, accept dye more readily than others. Other synthetic fibers can be colored during manufacture by tinting the polymeric material from which they are formed. In a further aspect of the invention, colored traces can be produced by exposing the braided suture material to a dye that is accepted by some strand materials and rejected by others. Those fibers that accept the dye become the colored trace, while fibers that reject the dye remain their original color, such as translucent or white.

In one embodiment, half of a length of suture is provided with tinted tracing fibers, or otherwise contrasts visually with the other half of the length of suture, which remains a plain, solid color, or displays a different tracing pattern, for example. Accordingly, when the length of suture is loaded through the eyelet of a suture anchor or passed through tissue, for example, at least one of the legs of the suture is visually coded, making identification and handling of the suture legs simpler. A few trace threads having a contrasting color, preferably of a readily dyed fiber such as polyester or nylon, in the cover aid surgeons in identifying the travel direction of the suture during surgery, particularly during arthroscopic operations and others, such as endoscopy and laparoscopy, that currently are generally referred to as "minimally invasive." Providing the trace threads in a regularly repeating pattern is particularly useful, allowing the surgeon to distinguish different ends of lengths of suture, and determine the direction of travel of a moving length of suture. Of the more easily dyed fibers, nylon is preferred in that it accepts dye readily.

In a preferred embodiment, the suture includes a multifilament jacket or sheath formed of braided PEEK. Optionally, the PEEK can be braided with an enhancement fiber from the group consisting of polyester, silk, nylon, ultrahigh molecular weight polyethylene and aramid, and combinations thereof. The jacket surrounds a core made substantially or entirely of PEEK. The core preferably includes three fibers of PEEK (144 decitex) twisted at about three to six twists per inch.

The jacket most preferably comprises eight fibers of PEEK (94 decitex yarn with 30 filaments) braided with six fibers of polyester (144 decitex). Optionally, one or more fibers of a material that can be dyed, such as nylon, can be provided in black or some other contrasting color as explained in greater detail below.

In another embodiment, the suture includes a multifilament jacket formed of ultrahigh molecular weight polyethylene fiber braided with PEEK. The jacket surrounds a core substantially or entirely of ultrahigh molecular weight polyethylene. The core preferably includes fibers of ultrahigh molecular weight polyethylene (144 decitex), twisted at about three to six twists per inch.

The jacket preferably includes eight fibers of ultrahigh molecular weight polyethylene (144 decitex) braided with eight fibers of PEEK (100 decitex yarn with 30 filaments) and two fibers of nylon, the nylon fibers being provided in black or some other contrasting color as explained in greater detail below.

The suture of the present invention advantageously has the strength of Ethibond No. 5 suture, yet has the diameter, feel, and tie-ability of No. 2 suture. As a result, the suture of the present invention is ideal for most orthopedic procedures such as rotator cuff repair, Achilles tendon repair, patellar tendon repair, ACL/PCL reconstruction, hip and shoulder reconstruction procedures, and replacement for suture used in or with suture anchors.

The suture can be uncoated or coated. Typically useful coatings include wax (beeswax, petroleum wax, polyethylene wax, or others), silicone (Dow Corning silicone fluid 202A or others), silicone rubbers (Nusil Med 2245, Nusil Med 2174 with a bonding catalyst, or others), PTFE (Teflon, Hostaflon, or others), PBA (polybutylate acid), ethyl cellulose (Filodel), and others known in the art. The coatings improve lubricity of the braid, and thus improve the handling characteristics, such as knot security, or abrasion resistance, for example.

As an added advantage, as mentioned above, some of the fibers in the cover may be provided in a contrasting color for visibility and identification purposes. A few trace threads having a contrasting color, preferably of a readily dyed fiber such as polyester or nylon, in the cover aid surgeons in identifying the travel direction of the suture during surgery, particularly during arthroscopic operations. Providing the trace threads in a regularly repeating pattern is particularly useful, allowing the surgeon to distinguish different ends of lengths of suture, and determine the direction of travel of a moving length of suture. Of the more easily dyed fibers, nylon is preferred in that it accepts dye readily.

Other features and advantages of the present invention will become apparent from the following description of the invention which refers to the accompanying drawings.

### BRIEF DESCRIPTION OF THE DRAWINGS

Fig. 1 is an enlarged detail view of a section of suture according to the present invention.

Fig. 2 is a schematic cross section of a length of suture according to the present invention.

Fig. 3 is an illustration of the suture of the present invention attached to a suture anchor loaded onto a driver.

Fig. 4A and 4B show the suture of the present invention attached to a half round, tapered needle.

Fig. 5 illustrates a bulk length of suture of the present invention.

Fig. 6 illustrates a strand of suture according to the present invention provided on a suture anchor.

### DETAILED DESCRIPTION OF THE INVENTION

The present invention is a high strength surgical suture material with surgically-useful qualities, including knot tie down characteristics and handling.

For purposes of simplicity and clarity, the term "PEEK," as used herein, is to be understood as including all variants of ether and ketone, including but not limited to, polyetherketone (PEK), polyetherketoneketone (PEKK), polyetherether-ketone (PEEK), and other ether and ketone variants.

The term "enhancement fiber," as used herein, is to be understood as including polyester, silk, nylon, ultrahigh molecular weight polyethylene and aramid, and combinations thereof.

The PEEK component of the present invention provides strength, and the enhancement fiber is provided to improve tie ability and tie down characteristics.

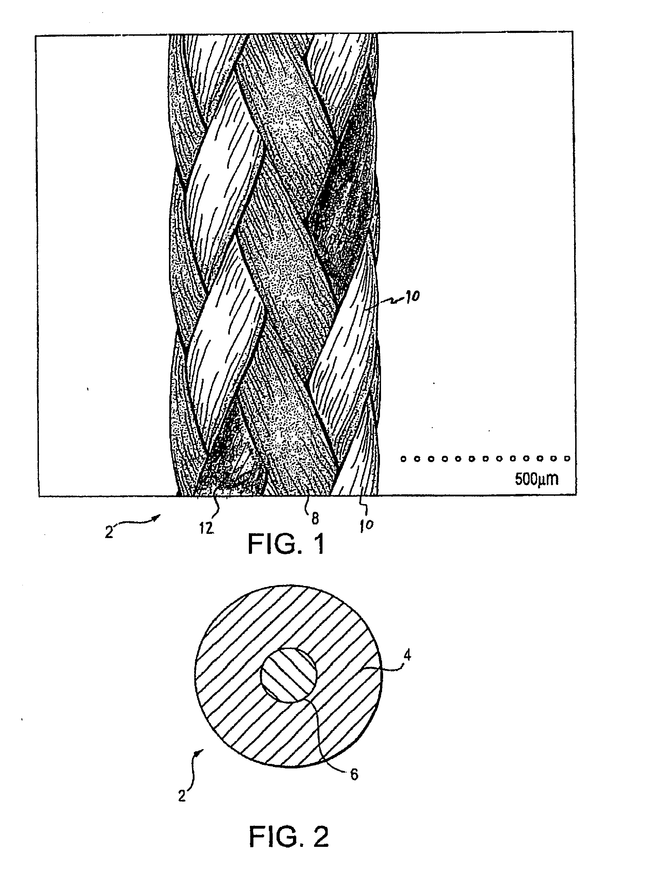

Referring now to the drawings, where like elements are designated by like reference numerals, Figs. 1 and 2 illustrate a section of suture 2 according to the present invention shown enlarged several fold. As illustrated in Fig. 2, suture 2 is made up of a jacket 4 and a core 6 surrounded by the jacket 4. Fibers of PEEK 8, optional fibers of enhancement fiber 10, and optional colored fibers 12 are braided together to form the jacket 4. Core 6 is formed of twisted fibers of PEEK or ultrahigh molecular weight polyethylene.

In accordance with the present invention, optional colored traces 12 are preferably black. The black trace assists surgeons in distinguishing between suture lengths with the trace and suture lengths without the trace. Traces also assist the surgeon in identifying whether or not, and in what direction, the suture is moving.
The trace can extend the entire length of the suture or only on half of a length of suture, the other half of the suture length remaining plain (white). Alternatively, the traces can form visibly distinct coding patterns on each half of the suture length. As a result, when the suture is threaded through the eyelet of a suture anchor, for example, the two legs (halves) of the length of suture are easily distinguished, and their direction of travel will be readily evident when the suture is pulled during surgery. Other patterns and arrangements of tracings also can be provided.

Ultrahigh molecular weight polyethylene fibers 8 are substantially translucent or colorless. All or the majority of the PEEK fibers 10 are white (undyed). Optionally, one or more PEEK or nylon fibers 12 may be provided in a contrasting color provide a trace in the suture. Due to the transparent nature of the ultrahigh molecular weight polyethylene, the suture takes on the color of fibers 10 and 12, and thus appears to be white with a trace in the contrasting color. In accordance with the present invention, trace fibers 12 are preferably provided in black. The black trace assists the surgeon in differentiating between suture strands with the trace and suture strands without the trace. The trace also assists the surgeon in identifying whether the suture is moving.

The colored fibers preferably are dyed filaments or fibers. Natural fibers, such as silk, and some synthetic fibers, accept dye more readily than others. Other synthetic fibers can be colored during manufacture by tinting the polymeric material from which they are formed. In a further aspect of the invention, colored traces can be produced by exposing the braided suture material to a dye that is accepted by some materials and rejected by others. Those fibers that accept the dye become the colored trace, while fibers that reject the dye remain their original color, such as translucent or white.

In one embodiment, half of a length of suture is provided with tinted tracing fibers, or otherwise contrasts visually with the other half of the length of suture, which remains a plain, solid color, or displays a different tracing pattern, for example. Accordingly, when the length of suture is loaded through the eyelet of a suture anchor or passed through tissue, for example, at least one of the legs of the suture is visually coded, making identification and handling of the suture legs simpler. Easy identification of suture *in situ* is advantageous in surgical procedures, particularly during arthroscopic surgeries and others, such as endoscopy and laparoscopy, that currently are generally referred to as "minimally invasive."

Details of the present invention will be described further below in connection with the following examples:

### EXAMPLE 1: USP Size 5 (EP size 7)

Made on a 16 carrier Hobourns machine, the yarns used in the hollow, plain braided jacket are PEEK, polyester type 712, and nylon. The jacket is formed using eight fibers of PEEK per carrier, braided with six fibers of 100 decitex polyester, and two fibers of tinted nylon. The core is formed of three carriers of PEEK braided at three to six twists per inch. A No. 5 suture is produced.

### EXAMPLE 2: Silk - Size 1

Core: 1 end of 144 decitex PEEK x 3

Jacket: 5 carriers 95 decitex polyester; 6 carriers 144 decitex PEEK; 1 carrier 84 decitex silk

The jacket is formed using six fibers of 144 decitex PEEK, braided with five fibers of 95 decitex polyester, and one strand of 84 decitex silk. The core is formed of three carriers of 144 decitex PEEK.

### EXAMPLE 3: Silk-Size 2

Core: 1 end of 144 decitex PEEK x 3

Jacket: 5 carriers 95 decitex polyester; 8 carriers 144 decitex PEEK; 1 carrier 84 decitex silk

The jacket is formed using eight fibers of 144 decitex PEEK, braided with five fibers of 95 decitex polyester, and one strand of 84 decitex silk. The core is formed of three carriers of 144 decitex PEEK.

### EXAMPLE 4:

The suture includes a multifilament jacket formed of ultrahigh molecular weight polyethylene fiber braided with PEEK. The cover surrounds a fiber core substantially or entirely of ultrahigh molecular weight polyethylene.

The core is formed using three fibers of 144 decitex ultrahigh molecular weight polyethylene, twisted at about three to six twists per inch. The jacket is formed using eight fibers of 144 decitex ultrahigh molecular weight polyethylene, braided with eight fibers of 94 decitex PEEK with thirty filaments.

### EXAMPLE 5:

The suture includes a multifilament jacket formed of ultrahigh molecular weight polyethylene fiber braided with PEEK. The jacket surrounds a fiber core substantially or entirely of ultrahigh molecular weight polyethylene.

The core is formed using a strand of 144 decitex ultrahigh molecular weight polyethylene, twisted at about three to six twists per inch. The jacket is formed using eight fibers of 144 decitex ultrahigh molecular weight polyethylene, braided with eight fibers of 100 decitex PEEK with thirty filaments.

### EXAMPLE 6:

The suture includes a multifilament jacket formed of ultrahigh molecular weight polyethylene fiber braided with PEEK. The jacket surrounds a fiber core substantially or entirely of ultrahigh molecular weight polyethylene.

The core is formed using a strand of 144 decitex ultrahigh molecular weight polyethylene, twisted at about three to six twists per inch. The jacket is formed using eight fibers of 144 decitex ultrahigh molecular weight polyethylene, braided with eight twisted fibers of PEEK.

As stated previously, one or more fibers of a fiber in the blend of any of the above examples can be provided in pre-dyed colors, e.g., black, to provide a trace. The trace threads enhance the ability to visually detect suture motion and the ability to differentiate between colored and uncolored suture strands.

To make various sizes of the inventive suture, different decitex values and different PPI settings can be used to achieve the required size and strength needed. In addition, smaller sizes may require manufacture on 12 carrier machines, for example. The very smallest sizes can be made without a core. Overall, the suture may range from 5% to 90% PEEK (preferably at least 40% of the fibers are PEEK), with the balance formed of enhancement fibers, such as polyester and/or silk. The core preferably comprises 18% or greater of the total amount of filament.

The suture preferably is coated with wax (beeswax, petroleum wax, polyethylene wax, or others), silicone (Dow Corning silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the braid, knot security, or abrasion resistance, for example.

According to an alternative embodiment of the present invention, a partially bioabsorbable suture is provided by blending PEEK fibers with a bioabsorbable material, such as PLLA or one of the other polylactides, for example. A suture made with about 10% PEEK blended with absorbable fibers would provide greater strength than existing bioabsorbable suture, and with less stretch. Over time, 90% or more of the suture would absorb, leaving only a very small remnant of the knot. The absorbable suture can include coatings and tinted traces as noted above for nonabsorbable suture.

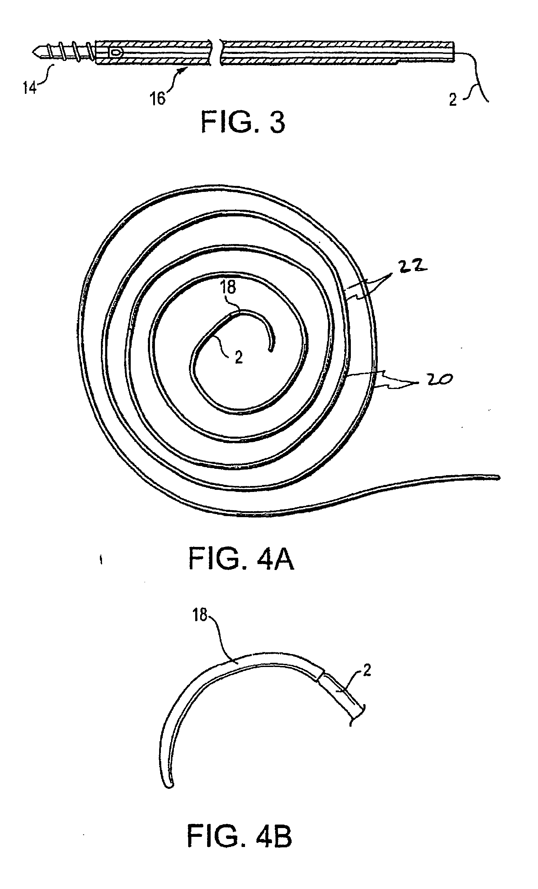

In one method of using the suture of the present invention, the suture 2 is attached to a suture anchor 14 as shown in Fig. 3 (prepackaged sterile with an inserter 16), or is attached at one or both ends to a half round, tapered needle 18 as shown in Figs. 4A and 4B. Fig. 4A also illustrates a length of suture having regularly repeating pattern of trace threads according to the present invention. Sections 20 of the length of suture 2 have tinted tracing threads woven in, while sections 22 of the length of suture are plain, or otherwise are distinguishable from sections 20. The alternating patterned and plain sections aid the surgeon in determining the direction of suture travel when pulling the suture through tissue as viewed through an arthroscope, for example.

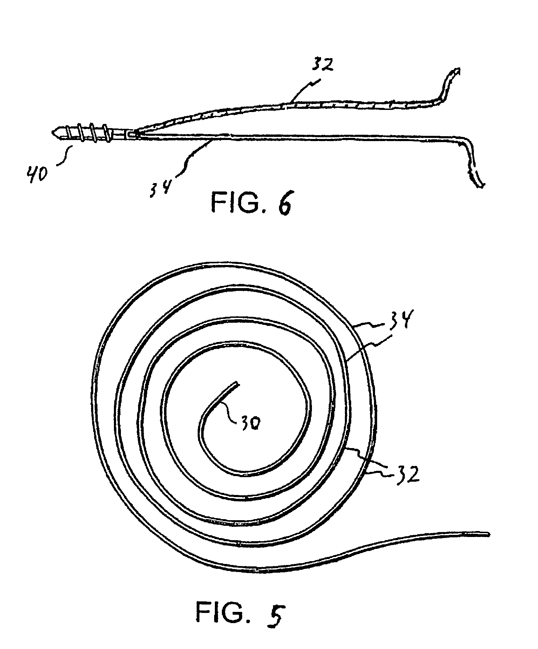

In yet another embodiment, as shown in Fig. 5, to make the suture which has a trace only at one end, bulk suture 30 is provided with repeating sections 32 having trace threads separated by sections 34 having no trace threads. The bulk suture is cut between every other section, at one end of each plain section, for example, to provide lengths of suture that are half traced and half plain. Alternatively, the bulk suture can be cut midway through each section to provide a shorter suture having a trace at one end.

In yet another embodiment, the half-and-half lengths of suture can be threaded through the eyelet of a suture anchor 40, as shown in Fig. 6. As a further alternative, uniform lengths of the braided suture can be exposed, partially or completely, to the dye (dipped, sprayed, etc.) to provide suture lengths with partial or complete dying patterns. Accordingly, the identity of each leg of the suture strand provided on the suture anchor is easily decoded by a surgeon operating with the suture anchor assembly.

Although the present invention has been described in relation to particular embodiments thereof, many other variations and modifications and other uses will become apparent to those skilled in the art. It is preferred, therefore, that the present invention be limited not by the specific disclosure herein, but only by the appended claims.

## Claims

1. A suture strand comprising a plurality of braided fibers formed of a polyether-ketone variant.

2. The suture strand of claim 1, further comprising a core comprising fibers of polyether-ketone variant surrounded by a jacket comprising fibers of polyether-ketone variant.

3. The suture strand of claim 1, wherein the fibers of polyether-ketone variant comprises at least 40% of the fibers in the suture strand.

4. The suture strand of claim 1, wherein the core comprises about 18% or greater of the total amount of fibers in the suture strand.

5. The suture strand of claim 2, wherein the core comprises braided fibers of polyether-ketone variant.

6. The suture strand of claim 1, wherein the polyether-ketone variant is selected from a group consisting of polyetherketone, polyetherketoneketone, and polyetheretherketone.

7. The suture strand of claim 1, further comprising a coating disposed on the jacket, the coating being selected from the group consisting of wax, silicone, silicone rubbers, PTFE, PBA, and ethyl cellulose.

8. The suture strand of claim 1, wherein the core is formed of at least three carriers of the polyether-ketone variant braided at three to six twists per inch.

9. The suture strand of claim 1, wherein the jacket is formed of fibers of the polyether-ketone variant braided with fibers of polyester.

10. The suture strand of claim 1, wherein the jacket is formed of fibers of the polyether-ketone variant braided with fibers of ultrahigh molecular weight polyethylene.

11. The suture strand of claim 1, wherein further comprising a core formed of twisted fibers of the polyether-ketone variant.

12. The suture strand of claim 1, wherein further comprising a core formed of twisted fibers of ultrahigh molecular weight polyethylene.

13. A suture assembly comprising:
a suture, the suture having a longitudinal length and a multifilament jacket comprising a plurality of braided fibers of polyether-ketone variant; and
a suture anchor, wherein the suture is threaded through an eyelet of the suture anchor.

14. A method of using a suture strand, comprising the steps of:
cutting a bulk length of multifilament suture material to make a plurality of suture fibers, the multifilament suture material comprising a plurality of braided fibers of polyether-ketone variant; and
attaching one of the plurality of suture strands to a suture anchor by threading through an eyelet of the suture anchor.

## Patentansprüche

1. Nahtfaden umfassend eine Mehrzahl von geflochtenen Fasern, die aus einer Polyether-Keton-Variante gebildet sind.

2. Nahtfaden nach Anspruch 1, außerdem umfassend einen Kern umfassend Fasern aus einer Polyether-Keton-Variante, der von einer Ummantelung umgeben ist, die Fasern aus einer Polyether-Keton-Variante umfasst.

3. Nahtfaden nach Anspruch 1, wobei die Fasern aus einer Polyether-Keton-Variante wenigstens 40 % der Fasern in dem Nahtfaden umfassen.

4. Nahtfaden nach Anspruch 1, wobei der Kern ungefähr 18 % oder mehr der Gesamtmenge an Fasern in dem Nahtfaden umfasst.

5. Nahtfaden nach Anspruch 2, wobei der Kern geflochtene Fasern aus einer Polyether-Keton-Variante umfasst.

6. Nahtfaden nach Anspruch 1, wobei die Polyether-Keton-Variante ausgewählt ist aus einer Gruppe bestehend aus Polyetherketon, Polyetherketonketon und Polyetheretherketon.

7. Nahtfaden nach Anspruch 1, außerdem umfassend eine auf der Ummantelung angeordnete Beschichtung, wobei die Beschichtung aus der aus Wachs, Silicon, Siliconkautschuken, PTFE, PBA und Ethylcellulose bestehenden Gruppe ausgewählt ist.

8. Nahtfaden nach Anspruch 1, wobei der Kern aus wenigstens drei Trägern aus der Polyether-Keton-Variante gebildet ist, die mit drei bis sechs Windungen pro Zoll geflochten sind.

9. Nahtfaden nach Anspruch 1, wobei die Ummantelung aus Fasern aus der Polyether-Keton-Variante, verflochten mit Fasern aus Polyester, gebildet ist.

10. Nahtfaden nach Anspruch 1, wobei die Ummantelung aus Fasern aus der Polyether-Keton-Variante, verflochten mit Fasern aus Polyethylen mit ultrahohem Moleklargewicht, gebildet ist.

11. Nahtfaden nach Anspruch 1, außerdem umfassend einen Kern, der aus verdrillten Fasern aus der Polyether-Keton-Variante gebildet ist.

12. Nahtfaden nach Anspruch 1, außerdem umfassend einen Kern, der aus verdrillten Fasern aus Polyethylen mit ultrahohem Molekulargewicht gebildet ist.

13. Nahtanordnung umfassend:
einen Nahtfaden, wobei der Faden eine longitudinale Länge und eine Multifilamentummantelung aufweist, die eine Mehrzahl von geflochtenen Fasern aus einer Polyether-Keton-Variante umfasst; und
einen Nahtanker, wobei der Faden durch eine Öse des Nahtankers eingefädelt ist.

14. Verfahren zum Verwenden eines Nahtfadens, umfassend die Schritte:
das Schneiden eines Multifilamentnahtmaterials, um eine Mehrzahl von Nahtfasern herzustellen, wobei das Multifilamentnahtmaterial eine Mehrzahl von geflochtenen Fasern aus einer Polyether-Keton-Variante umfasst; und
das Anbringen bzw. Befestigen von einem von der Mehrzahl von Nahtfäden an einem Nahtanker durch Einfädeln durch eine Öse des Nahtankers.

## Revendications

1. Fil de suture, comprenant une pluralité de fibres tressées formées d'un variant de polyéther-cétone.

2. Fil de suture suivant la revendication 1, comprenant en outre une âme comprenant des fibres d'un variant de polyéther-cétone entourée par une gaine comprenant des fibres d'un variant de polyéther-cétone.

3. Fil de suture suivant la revendication 1, dans lequel les fibres de variant de polyéther-cétone constituent au moins 40 % des fibres présentes dans le fil de suture.

4. Fil de suture suivant la revendication 1, dans lequel l'âme représente une proportion approximativement égale ou supérieure à 18 % de la quantité totale de fibres dans le fil de suture.

5. Fil de suture suivant la revendication 2, dans lequel l'âme comprend des fibres tressées de variant de polyéther-cétone.

6. Fil de suture suivant la revendication 1, dans lequel le variant de polyéther-cétone est choisi dans un groupe consistant en une polyéther-cétone, une polyéthercétonecétone, et une polyétheréther-cétone.

7. Fil de suture suivant la revendication 1, comprenant en outre un revêtement placé sur la gaine, le revêtement étant choisi dans le groupe consistant en une cire, une silicone, des caoutchoucs silicone, le PTFE, le PBA et l'éthylcellulose.

8. Fil de suture suivant la revendication 1, dans lequel l'âme est formée d'au moins trois supports du variant de polyéther-cétone tressés à raison de trois à six torsions par inch.

9. Fil de suture suivant la revendication 1, dans lequel la gaine est formée de fibres du variant de polyéther-cétone tressées avec des fibres de polyester.

10. Fil de suture suivant la revendication 1, dans lequel la gaine est formée de fibres du variant de polyéther-cétone tressées avec des fibres d'un polyéthylène de ultra-haut poids moléculaire.

11. Fil de suture suivant la revendication 1, qui comprend en outre une âme formée de fibres torsadées du variant de polyéther-cétone.

12. Fil de suture suivant la revendication 1, qui comprend en outre une âme formée de fibres torsadées d'un polyéthylène de ultra-haut poids moléculaire.

13. Assemblage comportant une suture, qui comprend :
une suture, la suture ayant une longueur dans la direction longitudinale et comprenant une gaine multifilaments comprenant une pluralité de fibres tressées d'un variant de polyéther-cétone ; et
un système d'ancrage de suture, dans lequel la suture est enfilée à travers un oeillet du système d'ancrage de suture.

14. Procédé d'utilisation d'un fil de suture, comprenant les étampes :
de coupe d'une grande longueur d'une matière de suture multifilaments pour préparer une pluralité de fibres de suture, la matière de suture multifilaments comprenant une pluralité de fibres tressées d'un variant de polyéther-cétone ; et
de fixation d'un fil de la pluralité de fils de suture à un système d'ancrage de suture par enfilage à travers un oeillet du système d'ancrage de suture.
